# EUROPEAN PATENT SPECIFICATION

(11) **EP 1 054 875 B1**
(45) Date of publication and mention of the grant of the patent: **23.04.2008**
(21) Application number: 99902412.8
(22) Date of filing: 22.01.1999
(51) Int. Cl.: C07D 273/04, C07D 285/16, C07D 253/06, C07D 413/04, C07D 498/04, C07F 9/6533, A01N 47/38, A01N 57/24

(54) **INSECTICIDAL DIHYDRO-OXADIAZINES, -THIADIAZINES AND -TRIAZINES**
INSEKTIZIDE DIHYDRO-OXADIAZINE, -THIADIAZINE UND -TRIAZINE
DIHYDRO-OXADIAZINES, -THIADIAZINES ET -TRIAZINES INSECTICIDES

(30) Priority: 12.02.1998 US 22616; 11.01.1999 US 228349
(43) Date of publication of application: 29.11.2000
(73) Proprietor: Chemtura Corporation, Middlebury, CT 06749 (US); Crompton Co./Cie, Elmira, Ontario N3B 3A3 (CA)
(72) Inventor: PARK, Sheldon, Bernard, Waterloo, Ontario N2K 3W7 (CA); MISHRA, Anupama, Guelph, Ontario N1G 2P7 (CA); DEKEYSER, Mark, Achiel, Waterloo, Ontario N2K 1W3 (CA); MCDONALD, Paul, Thomas, Middlebury, CT 06762 (US)
(74) Representative: Spott, Gottfried
(86) International application number: PCT/US1999/001245
(87) International publication number: WO 1999/041245

(56) References cited:
- WO-A-98/33794
- US-A- 3 397 201
- US-A- 3 420 826
- US-A- 5 536 720

## Description

### Field of the Invention

This invention relates to insecticidal substituted dihydrooxadiazine compounds, insecticidal compositions containing the dihydrooxadiazine compounds, and methods for their use.

### Background of the Invention

Certain oxadiazine compounds have been described as useful as pesticides and as pharmaceutical agents. For example, U.S. Patent No. 5,536,720 describes substituted 2-phenyl-1,3,4-oxadiazine-4-carbamide compounds useful as insecticides and acaricides. Trepanier et al, J. Med. Chem 9: 753-758 (1966) describe certain 2-substituted 4H-1,3,4-oxadiazines useful as anticonvulsants in mice. U.S. Patent No. 3,420,826 describes certain 2,4,6-substituted 4H-1,3,4-oxadiazines, useful as sedatives, anticonvulsants, and as pesticides against nematodes, plants, and fungi. U.S. Patent 3,420,825 describes methods for producing certain 2,4,6-substituted 4H-1,3,4-oxadiazines.

It is a purpose of this invention to provide novel dihydrooxadiazine derivatives useful as insecticides.

### Summary of the Invention

The present invention relates to a compound having the formula: 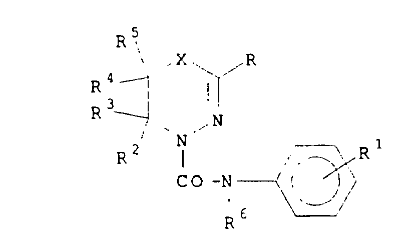 wherein
X is O, N or S;
R is phenyl, unsubstituted or mono-, di-, or tri-substituted with halogen, C₁-C₄ haloalkyl, C₁-C₄ haloalkoxy, or C₁-C₄ haloalkyl thio; or R is a C₄ -C₅ heterocyclic group containing one nitrogen, sulfur, or oxygen atom, unsubstituted or mono-, di-, or tri-substituted with halogen, C₁-C₄ haloalkyl, or C₁-C₄ haloalkoxy;
R¹ is halogen, C₁-C₄ haloalkyl, C₁-C₄ haloalkoxy, C₁-C₆ haloalkyl sulfonyl, or C₁-C₄ haloalkyl sulfinyl;
R², R³, R⁴, and R⁵ are one of the following:
a) R², R³, and R⁴ are, independently, hydrogen, C₁-C₆ alkyl, C₁-C₆ alkoxy, or furanyl; and R⁵ is nitro, cyano, C₁-C₆ alkyl, di(C₁-C₆)alkylamino, C₁-C₆ alkylthio, C₁-C₆ alkoxy, C₁-C₆ haloalkoxy, phenoxy, phenylthio, or (C₁-C₆ alkoxy) carbonyl, wherein R³ and R⁵ together can form a ring; or
b) R³, R⁴, and R⁵ are hydrogen; and R² is C₁-C₆ alkylthio, C₁-C₆ alkylsulfinyl, C₁-C₆ alkylsulfonyl, nitro(C₁-C₆ alkoxy), hydroxy, di-(C₁-C₄ alkoxy)-phosphinyl, cyano, C₂-C₆ acyloxy, C₁-C₆ alkoxy, C₁-C₆ haloalkoxy, C₄-C₆ cycloalkyl, or C₃-C₆ alkenyl; or R² is phenyl, phenylthio, phenylsulfinyl, phenylsulfonyl, pyrazolyl, furanyl, thienyl, phenyl(C₁-C₆ alkoxy), or benzoyloxy, unsubstituted or mono-, di-, or tri-substituted with halogen, C₁-C₄ alkyl, or C₁-C₄ alkoxy;
R⁶ is hydrogen, C₁-C₆ alkyl, C₁-C₄ alkylthio, (C₁-C₄ alkoxy)C₁-C₄ alkyl, C₂-C₈ acyl, benzyl, or (C₁-C₆ alkoxy)carbonyl.

These compounds, or physiologically acceptable salts thereof, are useful as insecticides.

The insecticidal compositions of this invention comprise: (a) an effective amount of one or more compounds of formula I, and (b) a suitable carrier.

### Detailed Description of the Invention

Preferably, the compound of this invention has the formula: 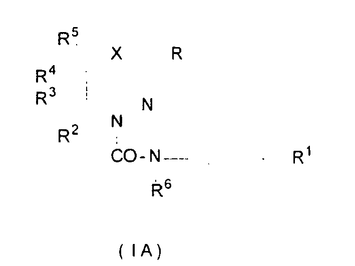 wherein R, R¹, R², R³, R⁴, R⁵, R⁶, and X are as described

In one preferred embodiment of this invention, R is phenyl, thienyl, furanyl, pyridinyl, optionally mono-, di- or tri-substituted by bromo, chloro, trihaloalkyl, or trihaloalkoxy, more preferably, one bromo, one chloro, one trihalomethyl, one trihaloethyl, one trihalomethoxy, or one trihaloethoxy; X is O; R¹ is C₁-C₄ trihaloalkyl or C₁-C₄ trihaloalkoxy, more preferably, trihalomethyl or trihalomethoxy; R², R³, and R⁴ are, independently, C₁-C₄ alkyl or C₁-C₄ alkoxy, more preferably, methyl, ethyl, methoxy, or ethoxy; R⁵ is C₁-C₄ alkoxy, C₁-C₄ alkyl, C₁-C₄ alkylthio, or di(C₁-C₄)alkylamino, more preferably, methoxy or ethoxy; and R⁶ is hydrogen, C₁-C₄ alkyl, C₁-C₄ alkylthio, (C₁-C₄ alkoxy) methyl or C₂-C₈ acyl, more preferably, methyl, methylthio, methoxymethyl or acetyl. Particularly preferred in this embodiment is the compound of formula IA wherein R is phenyl, thienyl, or pyridinyl, substituted by bromo, chloro, methyl, trifluoromethyl, or trifluoromethoxy.

In another preferred embodiment of this invention, the compound of this invention has the formula: 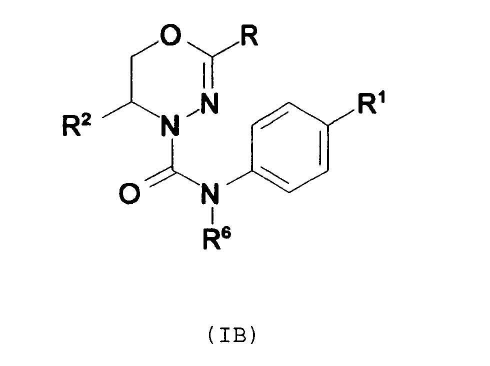 wherein
R is phenyl, unsubstituted or mono-, di-, or tri-substituted with halogen, C₁-C₄ haloalkyl, C₁-C₄ haloalkoxy, C₁-C₄ haloalkyl thio, C₁-C₄ haloalkyl sulfinyl, or C₁-C₄ haloalkyl sulfonyl; or R is a C₄-C₅ heterocyclic group containing one nitrogen, sulfur, or oxygen atom, unsubstituted or mono-, di-, or tri-substituted with halogen, C₁-C₄ haloalkyl, or C₁-C₄ haloalkoxy;
R¹ is halogen, C₁-C₄ haloalkyl, C₁-C₄, haloalkoxy, C₁-C₄ haloalkyl thio, C₁-C₆ haloalkyl sulfinyl, C₁-C₆ haloalkyl sulfonyl;
R² is C₁-C₆ alkylthio, C₁-C₆ alkylsulfinyl, C₁-C₆ alkylsulfonyl, nitro(C₁-C₆ alkoxy), hydroxy, di-(C₁-C₄ alkoxy)-phosphinyl, cyano, C₂-C₆ acyloxy, C₁-C₆ alkoxy, C₁-C₆ haloalkoxy, C₄-C₆ cycloalkyl, or C₃-C₆ alkenyl; or R² is phenyl, phenylthio, phenylsulfinyl, phenylsulfonyl, pyrazolyl, furanyl, thienyl, phenyl(C₁-C₆ alkoxy), or benzoyloxy, unsubstituted or mono-, di-, or tri-substituted with halogen, C₁-C₄ alkyl, or C₁-C₄ alkoxy; and
R⁶ is hydrogen, C₁-C₆ alkyl, (C₁-C₄ alkoxy) C₁-C₄ alkyl, C₂-C₆ haloacyl, C₂-C₈ acyl, or (C₁-C₆ alkoxy)carbonyl.

More preferred in this embodiment is the compound of IB wherein R is phenyl, optionally mono-, di- or tri-substituted by bromo, chloro, trihaloalkyl, or trihaloalkoxy, more preferably, one bromo, one chloro, one trihalomethyl, one trihaloethyl, one trihalomethoxy, or one trihaloethoxy; R¹ is C₁-C₄ trihaloalkyl or C₁-C₄ trihaloalkoxy, more preferably, trihalomethyl or trihalomethoxy; R² is C₁-C₆ alkylthio, C₁-C₆ alkylsulfinyl, C₁-C₆ alkylsulfonyl, nitro(C₁-C₄ alkoxy), hydroxy, di-(C₁-C₄ alkoxy)-phosphinyl, cyano, C₂-C₆ acyloxy, C₁-C₆ alkoxy, C,-C₆ haloalkoxy, C₄-C₆ cycloalkyl, or C₃-C₆ alkenyl, phenyl, phenylthio, phenylsulfinyl, phenylsulfonyl, pyrazolyl, mono- or di-(C₁-C₄ alkyl)-pyrazolyl, furanyl, (C₁-C₄ alkyl)furanyl, thienyl, phenyl(C₁-C₄ alkoxy), or benzoyloxy; and R⁶ is hydrogen, C₁-C₄ alkyl, C₁-C₄ alkylthio, (C₁-C₄ alkoxy)methyl or C₂-C₈ acyl, more preferably, hydrogen, methyl, methylthio, methoxymethyl or acetyl. Particularly preferred in this embodiment is the compound of formula IB wherein R is phenyl substituted by bromo, chloro, methyl, trifluoromethyl, or trifluoromethoxy.

The compounds and compositions of this invention are useful as plant protecting agents against insects and are particularly effective against coleopterous insects and lepidopterous insects, such as tobacco budworm.

The compounds of the instant invention can be prepared by reacting an oxadiazine of formula A below, wherein R, R², R³, R⁴, R⁵, and X are as described above, with an isocyanate of formula B below, wherein R¹ is as described above, and a catalytic amount of triethylamine in a suitable solvent such as acetonitrile or toluene, to produce the compound of formula IC (the compounds of formula I in which R⁶ is hydrogen) .

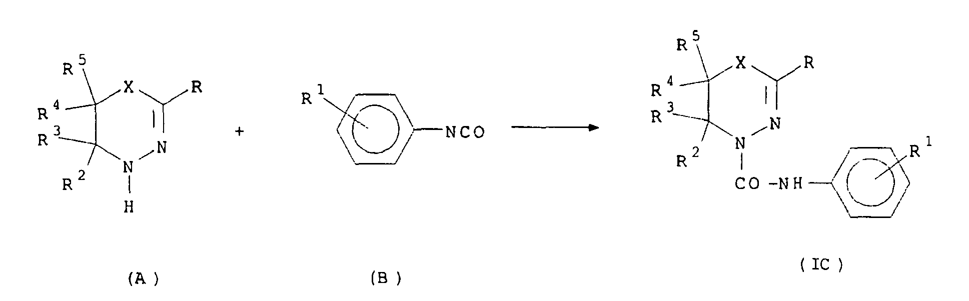

The compounds of formula I in which R⁶ is C₁-C₆ alkyl, C₁-C₄ alkylthio, (C₁-C₄ alkoxy)C₁-C₄ alkyl, C₂-C₈ acyl, or benzyl, can be prepared by reacting the compound of formula IB with R⁶Y wherein Y is halogen and R⁶ is C₁-C₆ alkyl, C₁-C₄ alkylthio, (C₁-C₄ alkoxy) C₁-C₄ alkyl, C₂-C₈ acyl, or benzyl, with a suitable base such as triethylamine or sodium hydride.

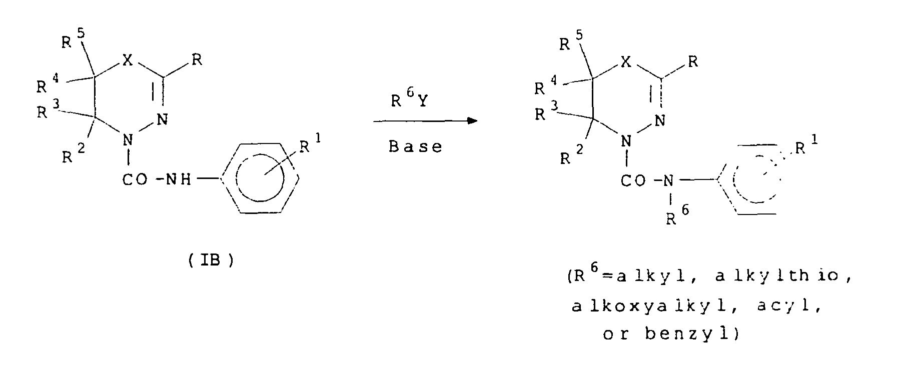

Compounds of formula A above can be prepared by the cyclization of an azo compound (RCXN=NCO₂C₂H₅) with an alkene of the formula: 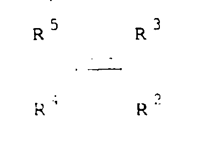 wherein R, R², R³, R⁴, R⁵, and X are as described above,
followed by hydrolysis of the resultant intermediate.

The compounds of formula IB in which R³, R⁴, and R⁵ are hydrogen; and R² is C₁-C₆ alkylthio, C₁-C₆ alkylsulfinyl, C₁-C₆ alkylsulfonyl, nitro(C₁-C₆ alkoxy), hydroxy, di-(C₁-C₄ alkoxy) -phosphinyl, cyano, C₂-C₆ acyloxy, C₁-C₆ alkoxy, C₁-C₆ haloalkoxy, C₄-C₆ cycloalkyl, or C₃-C₆ alkenyl; or R² is phenyl, phenylthio, phenylsulfinyl, phenylsulfonyl, pyrazolyl, furanyl, thienyl, phenyl (C₁-C₆ alkoxy), or benzoyloxy, unsubstituted or mono-, di-, or tri-substituted with halogen, C₁-C₄ alkyl, or C₁-C₄ alkoxy, can be prepared by reacting an oxadiazine of formula C below, wherein R, R¹, and R⁶ are as described above, with a nucleophile R²H, wherein R² is as described above, and a Lewis acid such as boron trifluoride etherate in a suitable solvent such as dichloromethane or tetrahydrofuran to produce the compound of formula IB.

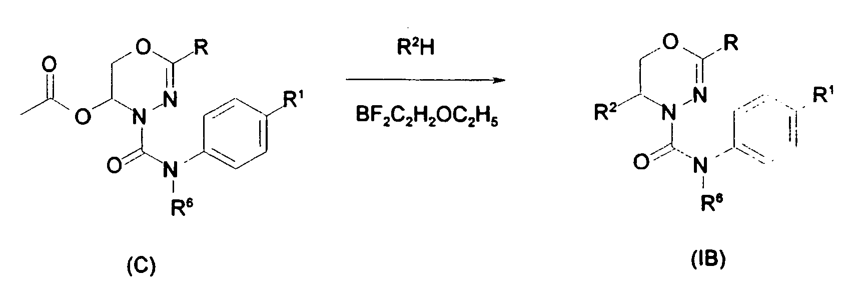

Compounds of formula (C) above can be prepared by the following scheme in which an oxadiazinone of formula (II) is reacted with an isocyanate of formula (III) and a catalytic amount of tributyltin diacetate in a solvent such as acetonitrile to give the intermediate (IV) which is further reacted with a reducing agent sodium borohydride to give the intermediate alcohol (V) which is further reached with an acid chloride such as acetyl chloride to give the intermediate (C). The compounds of formula IA in which R² is hydroxy or acyloxy or aroyloxy are made at the intermediate stages shown in the scheme:

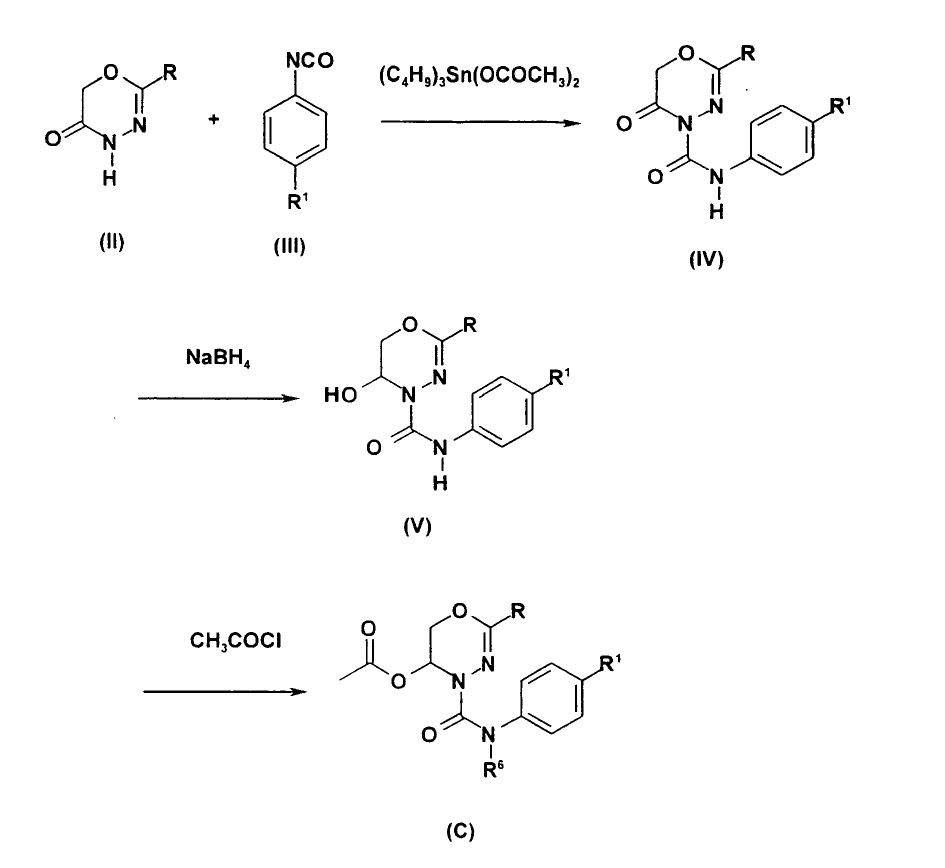

Some of the compounds of formula IB in which R is alkylthio or arylthio can be further reacted to give the alkylsulfinyl, alkylsulfonyl, arylsulfinyl or arylsulfonyl derivatives by oxidation with meta-chloroperbenzoic acid.

The compositions of the present invention can be prepared by formulating one or more compounds of the present invention with a suitable carrier.

Suitable liquid carriers can comprise water, alcohols, ketones, phenols, toluene and xylenes. In such formulations, additives conventionally employed in the art can be utilized, such as one or more surface active agents and/or inert diluents, to facilitate handling and application of the resulting insecticidal composition.

Alternatively, the compounds of this invention can be applied as a liquid or in sprays when utilized in a liquid carrier, such as a solution comprising a compatible solvent such as acetone, benzene, toluene or kerosene, or a dispersion comprising a suitable non-solvent medium such as water.

The compositions of this invention can alternatively comprise solid carriers taking the form of dusts, granules, wettable powders, pastes, aerosols, emulsions, emulsifiable concentrates, and water-soluble solids. For example, the compounds of this invention can be applied as dusts when admixed with or absorbed onto powdered solid carriers, such as mineral silicates, talc, pyrophyllite and clays, together with a surface-active dispersing agent so that a wettable powder is obtained which then is applied directly to the loci to be treated. Alternatively, the powdered solid carrier containing the compound admixed therewith, can be dispersed in water to form a suspension for application in such form.

Granular formulations of the compounds are preferred for field treatment and are suitable for application by broadcasting, side dressing, soil incorporation or seed treatment, and are suitably prepared using a granular or pelletized form of carrier such as granular clays, vermiculite, charcoal or corn cobs. The compound of this invention is dissolved in a solvent and sprayed onto an inert mineral carrier such as attapulgite granules (10-100 mesh), and the solvent is then evaporated. Such granular compositions can contain from 2-25% of a compound of this invention, based on carrier plus compound, preferably, 3-15%. In addition, the compounds of this invention can also be incorporated into a polymeric carrier such as polyethylene, polypropylene, butadiene-styrene, styrene-acrylonitrile resins, polyamides, poly(vinyl acetates), and the like. When encapsulated, the compound of this invention can advantageously be released over an even longer time period, extending its effectiveness further than when used in non-encapsulated form.

Another method of applying the compound of this invention to the loci to be treated is by aerosol treatment, for which the compound can be dissolved in an aerosol carrier which is a liquid under pressure but which is a gas at ordinary temperature (e.g., 20°C) and atmospheric pressure. Aerosol formulations can also be prepared by first dissolving the compound in a less volatile solvent and then admixing the resulting solution with a highly volatile liquid aerosol carrier.

For treatment of plants (such term including plant parts), the compounds of the invention preferably are applied in aqueous emulsions containing a surface-active dispersing agent which can be non-ionic, cationic or anionic. Suitable surface-active agents are well known in the art, such as those disclosed in U.S. Patent 2,547,724 (columns 3 and 4). The compounds of this invention can be mixed with such surface-active dispersing agents, with or without an organic solvent, as concentrates for the subsequent addition of water, to yield aqueous suspensions of the compounds at desired concentration levels.

In addition, the compounds can be employed with carriers which themselves are pesticidally active, such as insecticides, acaricides, fungicides or bactericides.

It will be understood that the effective amount of a compound in a given formulation will vary depending, e.g., upon the specific pest to be combated, as well as upon the specific chemical composition and formulation of the compound being employed, the method of applying the compound/formulation and the locus of treatment. Generally, however, the effective amount of the compound of this invention can range from about 0.1 to about 95 percent by weight. Spray dilutions can be as low as a few parts per million, while at the opposite extreme, full strength concentrates of the compound can be usefully applied by ultra low volume techniques. When plants constitute the loci of treatment, concentration per unit area can range between about 0.01 and about 50 pounds per acre, with concentrations of between about 0.1 and about 10 pounds per acre preferably being employed for crops such as corn, tobacco, rice and the like.

To combat insects, sprays of the compounds can be applied to any suitable locus, such as to the insects directly and/or to plants upon which they feed or nest. The compositions of this invention can also be applied to the soil or other medium in which the pests are present.

The specific methods of application of the compounds and compositions of this invention, as well as the selection and concentration of these compounds, will vary depending upon such circumstances as crops to be protected, geographic area, climate, topography, plant tolerance, etc.

The following examples are provided to illustrate the present invention.

### EXAMPLES

### Example 1

### Preparation of 6-ethoxy-5,6-dihydro-N-[4-(trifluoromethoxy)phenyl]-2-[3-(trifluoromethyl)phenyl]-4H-1,3,4-oxadiazine-4-carboxamide (Compound No. 1)

### A. Preparation of 2-[3-(trifluoromethyl)benzoyl]-hydrazinecarboxylic acid ethyl ester

70g of (3-trifluoromethyl)benzoic acid hydrazide (0.34 mol) and 40 ml of pyridine were dissolved in 150 ml of N,N-dimethylformamide and stirred at 15°C (water bath). 40 ml of ethyl chloroformate (0.41 mol) was added dropwise to this mixture while the reaction temperature was kept below 20°C. After addition of the ethylchloroformate, the resultant reaction mixture was stirred at room temperature for four hours and then 500 ml of water was added with stirring. The resultant precipitate was filtered by suction, washed with water four times, and then dried in air, to produce 80g of 2-[3-(trifluoromethyl)benzoyl]hydrazinecarboxylic acid ethyl ester (85% yield). The structure was confirmed by infrared (IR) and nuclear magnetic resonance (NMR) spectra.

### B. Preparation of 4-[3-trifluoromethyl)benzoyl]-diazenecarboxylic acid ethyl ester

15g of 2-[3-(trifluoromethyl)benzoyl]hydrazinecarboxylic acid ethyl ester was stirred with 200 ml of dichloromethane at room temperature. 150 ml of 5% NaOCl (0.10 mol) solution was then rapidly added to the reaction mixture, which exothermed to 30°C, and stirred for three hours until all the white solid disappeared and a deep red colored organic layer appeared. This organic layer was separated, dried (MgSO₄), and the solvent removed to give 13.5g of 4-[3-trifluoromethyl)benzoyl]-diazenecarboxylic acid ethyl ester (89%). The structure was confirmed by IR and NMR spectra.

### C. Preparation of 6-ethoxy-5,6-dihydro-2-[3-(trifluoromethyl)phenyl]-4H-1,3,4-oxadiazine-4-carboxylic acid ethyl ester

20g of benzene was added to 13.5g of 4-[3-trifluoromethyl)benzoyl]-diazenecarboxylic acid ethyl ester, followed by the addition of 7g of ethyl vinyl ether to prepare a reaction mixture. The reaction mixture was kept at room temperature overnight. After the red color of the reaction mixture faded, the solvent was removed, giving 17g of crude 6-ethoxy-5,6-dihydro-2-[3-(trifluoro-methyl)phenyl]-4H-1,3,4-oxadiazine-4-carboxylic acid ethyl ester, which was not purified and was used in Step D below.

### D. Preparation of 6-ethoxy-5,6-dihydro-2-[3-(trifluoromethyl)phenyl]-4H-1,3,4-oxadiazine

The crude 6-ethoxy-5,6-dihydro-2-[3-(trifluoromethyl)phenyl]-4H-1,3,4-oxadiazine-4-carboxylic acid ethyl ester (17g) from Step C above, was mixed with 12g of KOH, 20g of ethanol, and 40g of water to create a reaction mixture. The reaction mixture was refluxed for 6 hours and then cooled. 50 ml of water was then added to the reaction mixture, followed by extraction with ethyl acetate (4 x 100 ml). The ethyl acetate extracts were conbined, dried (MgSO₄) and the solvent removed, to give 7g of crude 6-ethoxy-5,5-dihydro-2-[3-(trifluoromethyl)phenyl]-4H-1,3,4-oxadiazine, which was not purified and was used in Step E below.

### E. Preparation of 6-ethoxy-5,6-dihydro-N-[4-(trifluoromethoxy)phenyl] -2- [3-trifluoromethyl)phenyl] -4H-1,3,4-oxadiazine-4-carboxamide (Compound No. 1)

The crude 6-ethoxy-5,6-dihydro-2-[3-(trifluoromethyl)phenyl]-4H-1,3,4-oxadiazine (7g) from Step D above, was dissolved in 20g of acetonitrile and then 5g of (4-trifluoromethoxy)phenyl isocyanate was added, to create a reaction mixture. The reaction mixture was refluxed for four hours and then the acetonitrile was removed. The residue was then purified by column chromatography on 100g of silica gel using 1000 ml of toluene. The toluene was removed and the residue was crystallized with methanol to give two crops of crystals, total weight 5g (0.015 mol) of 6-ethoxy-5,6-dihydro-N-[4-(trifluoro-methoxy)phenyl]-2-[3-trifluoromethyl)phenyl]-4H-1,3,4-oxadiazine-4-carboxamide (26% yield based on hydrazinecarboxylic acid ethyl ester), m.p. 105-107°C.

### Example 2

### Preparation of 6-ethoxy-5,6-dihydro-N-[4-(trifluoromethoxy)phenyl]-2-(4-bromophenyl)-4H-1,3,4-oxadiazine-4-carboxamide (Compound No. 2)

Prepared as described above in Example 1 except that 4-bromobenzoic acid hydrazide was used instead of 3-trifluoromethyl benzoic acid hydrazide in Step A.

### Example 3

### Preparation of 6-ethoxy-5,6-dihydro-N-[4-(trifluoromethoxy)phenyl]-2-(5-bromo-2-thiophenyl)-4H-1,3,4-oxadiazine-4-carboxamide (Compound No. 3)

Prepared as described above in Example 1 except that 5-bromo-2-thiophenecarboxylic acid hydrazide was used instead of 3-trifluoromethyl benzoic acid hydrazide in Step A.

### Example 4

### Preparation of 6-methoxy-6-methyl-5-hydro-N-[4-(trifluoro-methoxy)phenyl]-2-(5-bromo-2-thiophenyl)-4H-1,3,4-oxadiazine-4-carboxamide (Compound No. 4)

Prepared as described above in Example 1 except that 5-bromo-2-thiophenecarboxylic acid hydrazide was used instead of 3-trifluoromethyl benzoic acid hydrazide in Step A and 2-methoxypropene was used instead of ethyl vinyl ether in Step C.

### Example 5

### Preparation of 5-methyl-6-ethoxy-5,6-dihydro-N-[4-(trifluoromethoxy)phenyl]-2-[3-(trifluoromethyl)phenyl]-4H-1,3,4-oxadiazine-4-carboxamide (Compound No. 5)

Prepared as described above in Example 1 except that ethyl-1-propenyl ether was used instead of ethyl vinyl ether in Step C.

### Example 6

### Preparation of (2,3-dihydrofuranyl)-N-[4-(trifluoromethoxy)phenyl]-2-(4-bromophenyl)-4H-1,3,4-oxadiazine-4-carboxamide (Compound No. 6)

Prepared as described above in Example 1 except that 4-bromobenzoic acid hydrazide was used instead of 3-trifluoromethyl benzoic acid hydrazide in Step A and 2,3-dihydrofuran was used instead of ethyl vinyl ether in Step C.

### Example 7

### Preparation of (3,4-dihydropyranyl)-N-[4-(trifluoromethoxy)phenyl]-2-(4-bromophenyl)-4H-1,3,4-oxadiazine-4-carboxamide (Compound No. 7)

Prepared as described above in Example 1 except that 4-bromobenzoic acid hydrazide was used instead of 3-trifluoromethyl benzoic acid hydrazide in Step A and 3,4-dihydropyran was used instead of ethyl vinyl ether in Step C.

### Example 8

### Preparation of 6-methoxy-5,6-dihydro-N-[4-trifluoromethoxy)phenyl]-2-[3-(trifluoromethyl)phenyl]-4H-1,3,4-oxadiazine-4-carboxamide (Compound No. 8)

Prepared as described above in Example 1 except that methyl vinyl ether was used instead of ethyl vinyl ether in Step C.

### Example 9

### Preparation of 6-ethoxy-5,6-dihydro-N-[4-(trifluoromethyl)phenyl]-2-[3-(trifluoromethyl)phenyl]-4H-1,3,4-oxadiazine-4-carboxamide (Compound No. 9)

Prepared as described above in Example 1 except that (4-trifluoromethyl)phenyl isocyanate was used instead of (4-trifluoromethoxy)phenyl isocyanate in Step E.

### Example 10

### Preparation of 5,6-dihydro-6-methyl-N-[4-(trifluoromethyl)phenyl]-2-[4-(trifluoromethoxy)phenyl]-4H-1,3,4-oxadiazine-4-carboxamide (Compound No. 10)

### A. Preparation of 2-(2-chloro-1-oxopropyl)hydrazide of 4-(trifluoromethoxy)benzoic acid

11g of 4-(trifluoromethoxy)benzoic acid hydrazide (0.05 mol) and 6.5 g of 2-chloropropionyl chloride (0.05 mol) were dissolved in 100 ml of 1,4-dioxane and stirred at reflux for three hours. After evaporation of the solvent under reduced pressure, the remaining oil was purified by column chromatography. 8 g of 2-(2-chloro-1-oxopropyl)hydrazide of 4-(trifluoromethoxy)benzoic acid as a white solid was obtained. The structure was confirmed by nuclear magnetic resonance spectroscopy.

### B. Preparation of 6-methyl-2-(4-trifluoromethoxy)-phenyl-4H-1,3,4-oxadiazin-5(6H)-one.

8 g of the 2-(2-chloro-1-oxopropyl)hydrazide of 4-(trifluoromethoxy)benzoic acid (0.03 mol) prepared above in A was dissolved in 100 ml of acetonitrile. 3 g of triethylamine was then added and the resulting solution was stirred and refluxed for 24 hours. After cooling, the precipitate was filtered and the filtrate evaporated under reduced pressure, leaving 7.5 g of an oil. Purification by column chromatography produced 3 g of 6-methyl-2-(4-trifluoromethoxy)-phenyl-4H-1,3,4-oxadiazin-5(6H)-one as a white solid. The structure was confirmed by nuclear magnetic resonance spectroscopy.

### C. Preparation of 5,6-dihydro-6-methyl-2-(4-trifluoromethoxyphenyl)-4H-1,3,4-oxadiazine.

3 g of the 6-methyl-2-(4-trifluoromethoxy)phenyl-4H-1,3,4-oxadiazin-5(6H)-one prepared above in B was dissolved in 25 ml of 1,4-dioxane. The solution was cooled to 0°C and then 0.5 g of acetic acid was added. The reaction mixture was then refluxed for 24 hours. After cooling to room temperature, 100 ml of water was added and the mixture was extracted with 200 ml of dichloromethane. 2.6 g of an oil remained which was purified by column chromatography to produce 1.0 g of 5,6-dihydro-6-methyl-2-(4-trifluoromethoxyphenyl)-4H-1,3,4-oxadiazine as an oil. The structure was confirmed by nuclear magnetic resonance spectroscopy.

### D. Preparation of 5,6-dihydro-6-methyl-2-(4-trifluoromethoxyphenyl)-N-(4-trifluoromethylphenyl)-4H-1,3,4-oxadiazine-4-carboxamide.

1 g of the 5,6-dihydro-6-methyl-2-(4-trifluoromethoxyphenyl)-4H-1,3,4-oxadiazine prepared above was dissolved in 10 ml of acetonitrile. 1 g of 4-(trifluoromethyl)phenyl isocyanate was added dropwise and then the resulting reaction mixture was refluxed for two hours. Evaporation of the solvent at reduced pressure afforded 1.5 g of 5,6-dihydro-6-methyl-2-{4-trifluoromethoxyphenyl)-N-(4-triflueromethylphenyl)-4H-1,3,4-oxadiazine-4-carboxamide as a solid which was washed with a few milliliters of acetonitrile. The structure was confirmed by nuclear magnetic resonance spectroscopy.

### Example 11

### Preparation of ethyl 5,6-dihydro-2-[4-(trifluoromethoxy)-phenyl]-N-[((4-trifluoromethylphenyl)amino)carbonyl]-4H-1,3,4-oxadiazine-6-carboxylate (Compound No. 11)

### A. Preparation of ethyl 5,6-dihydro-2-(4-trifluoromethoxyphenyl-4H-1,3,4-oxadiazine-6-carboxylate.

25 g of 4-(trifluoromethoxy)benzoic acid hydrazide (0.13 mol) and 30 g of ethyl 2,3-dibromopropionate (0.13 mol) were dissolved in 150 ml of acetonitrile. 26 g (0.26 mol) of triethylamine was then added and then the resultant reaction mixture was refluxed for 24 hours. After cooling, the reaction mixture was filtered and then the filtrate was evaporated under reduced pressure leaving 35 g of a crude oil. The crude oil was purified by column chromatography to afford 4.5 g of ethyl 5,6-dihydro-2-(4-trifluoro-methoxyphenyl-4H-1,3,4-oxadiazine-6-carboxylate as an oil. The structure was confirmed by nuclear magnetic resonance spectroscopy.

### B. Preparation of ethyl 5,6-dihydro-2-(4-trifluoromethoxyphenyl)-N-(((4-trifluoromethylphenyl)amino)-carbonyl)-4H-1,3,4-oxadiazine-6-carboxylate.

1 g of the ethyl 5,6-dihydro-2-(4-trifluoromethoxyphenyl-4H-1,3,4-oxadiazine-6-carboxylate prepared above in A was dissolved in 15 ml of acetonitrile. 1 g of 4-(trifluoromethyl)phenyl isocyanate was added dropwise. The resulting reaction mixture was refluxed for two hours and then the solvent was evaporated under reduced pressure to produce a solid. The solid was washed with a few milliliters of acetonitrile to produce 1.2 g of ethyl 5,6-dihydro-2-(4-trifluoromethoxyphenyl)-N-(((4-trifluoromethylphenyl)aminocarbonyl)-4H-1,3,4-oxadiazine-6-carboxylate as a white solid. The structure was confirmed by nuclear magnetic resonance spectroscopy.

### Example 12

### Preparation of 4H-1,3,4-oxadiazine-4-carboxamide, 6-ethoxy-5,6-dihydro-N-methyl-N-[4-(trifluoromethoxy)-phenyl]-2-[3-(trifluoromethyl)phenyl]-(Compound No. 12)

1g of 6-ethoxy-5,6-dihydro-N-[4-(trifluoromethoxy)phenyl]-2-[3-trifluoromethyl)phenyl]-4H-1,3,4-oxadiazine-4-carboxamide (Compound No. 1) was added to 0.3g of hexane-washed sodium hydride dissolved in 20 ml of toluene and the resulting reaction mixture was heated to 60°C for one hour. After to room temperature, 3 ml of methyl iodide was added dropwise to the reaction mixture. The reaction mixture was then stirred at room temperature for 18 hours. 100 ml of water was then added to the reaction mixture and then extracted with 100 mol of toluene. After drying over sodium sulfate, the filtered solution was evaporated under reduced pressure, to produce an oil. The oil was purified by column chromatography producing 6-ethoxy-5,6-dihydro-N-methyl-N-[4-(trifluoromethoxy)phenyl]-2-[3-(trifluoromethyl)-phenyl]-4H-1,3,4-oxadiazine-4-carboxamide, as a viscous oil. The structure was confirmed by nuclear magnetic resonance spectroscopy.

### Example 13

### Preparation of 5-(butylthio)-5,6-dihydro-2-[3-(trifluoromethyl)phenyl]-N-[4-(trifluoromethyl)phenyl]-4H-1,3,4-oxadiazine-4-carboxamide (Compound No. 14)

### A. Preparation of 2-[3-(trifluoromethyl)phenyl]-4H-1,3,4-oxadiazin-5(6H)-one

39.0 ml of chloroacetyl chloride was added to a solution of 100 g of 3-trifluoromethylbenzoic acid hydrazide in 500 ml 1,4-dioxane and the resultant mixture was heated to reflux for 1.5 h. The mixture was then cooled and concentrated under vacuum to produce a residue. The residue was suspended in 21 acetonitrile and then 200 ml triethylamine was added. The resultant mixture was heated to reflux for 16 h and then cooled and filtered. The filtrate was concentrated under vacuum to give crude 2-[3-(trifluoromethyl)phenyl]-4H-1,3,4-oxadiazin-5(6)-one which was purified by recrystallization from ethanol/water. The structure was confirmed by 1H NMR.

### B. Preparation of 5,6-Dihydro-5-oxo-2-[3-(trifluoromethyl)phenyl]-N-[4-(trifluoro-methyl)phenyl]-4H-1,3,4-oxadiazine-4-carboxamide

A mixture of 52.5 g 2-[3-(trifluoromethyl)phenyl]-4H-1,3,4-oxadiazin-5(6)-one (prepared in A above), 52.5 g 4-trifluoromethyl-phenyl isocyanate and 2 ml dibutyltin diacetate in 500 ml acetonitrile was heated to reflux for 3 h. The mixture was then cooled and filtered to produce 5,6-dihydro-5-oxo-2-[3-(trifluoro-methyl)phenyl]-N-[4-(trifluoromethyl)phenyl]-4H-1,3,4-oxadiazine-4-carboxamide as a white solid. The structure was confirmed by 1H NMR.

### C. Preparation of 5,6-Dihydro-5-hydroxy-2-[3-(trifluoromethyl)phenyl]-N-[4-(trifluoromethyl)-phenyl]-4H-1,3,4-oxadiazine-4-carboxamide

To an ice-cooled suspension of 26.0 g 5,6-dihydro-5-oxo-2-[3-(trifluoromethyl)phenyl]-N-[4-(trifluoromethyl)-phenyl]-4H-1,3,4-oxadiazine-4-carboxamide (prepared in B above) in 300 ml dichloromethane and 300 ml methanol was added 2.3 g sodium borohydride in portions. The resultant mixture was stirred for 45 min and then quenched with 200 ml 1M HCl and diluted with 1 1 dichloromethane. The phases were separated and the organic phase was washed with 200 ml 1M HCl and 100 ml saturated sodium bicarbonate, dried over sodium sulfate and concentrated under vacuum to give 5,6-dihydro-5-hydroxy-2-[3-(trifluoromethyl)phenyl]-N-[4- (trifluoromethyl)phenyl]-4H-1,3,4-oxadiazine-4-carboxamide. The structure was confirmed by 1H NMR.

### D. Preparation of 5,6-dihydro-5-acetyloxy-2-[3-(trifluoro-methyl)phenyl]-N-[4-(trifluoromethyl)-phenyl]-4H-1,3,4-oxadiazine-4-carboxamide

A mixture of 21.0 g 5,6-dihydro-5-hydroxy-2-[3-(trifluoromethyl)phenyl]-N-[4-(trifluoromethyl)phenyl]-4H-1,3,4-oxadiazine-4-carboxamide (prepared in C above), 13.0 ml triethylamine, and 6.5 ml acetic anhydride was stirred at room temperature for 3 h. The mixture was then concentrated under vacuum to produce a solid residue. The solid residue was taken up in 400 ml dichloromethane and washed twice with 100 ml saturated sodium bicarbonate. The organic phase was then dried over sodium sulfate and concentrated under vacuum to produce 5,6-dihydro-5-acetyloxy-2-[3-(trifluoromethyl)phenyl] -N- [4-(trifluoromethyl)phenyl]-4H-1,3,4-oxadiazine-4-carboxamide. The structure was confirmed by 1H NMR.

### E. Preparation of 5-(butylthio)-5,6-dihydro-2-[3-(trifluoromethyl)phenyl]-N-[4-(trifluoromethyl)-phenyl]-4H-1,3,4-oxadiazine-4-carboxamide

To a cooled (-78 °C) mixture of 3.0 g 5,6-dihydro-5-acetyloxy-2-[3-(trifluoromethyl)phenyl]-N-[4-(trifluoromethyl)phenyl]-4H-1,3,4-oxadiazine-4-carboxamide (prepared in D above) and 2.0 ml butanethiol in 50 ml dichloromethane was added 1.0 ml borontrifluoride etherate. The cooling bath was removed and the mixture was stirred for 1 h. The mixture was then quenched with saturated sodium bicarbonate and the phases were separated. The organic phase was washed with 1 M sodium hydroxide and saturated sodium bicarbonate, dried over sodium sulfate and concentrated under vacuum to give 5-(butylthio)-5,6-dihydro-2-[3-(trifluoromethyl)phenyl]-N-[4-(trifluoromethyl)phenyl]-4H-1,3,4-oxadiazine-4-carboxamide. The structure was confirmed by 1H NMR.

### Example 14

### Preparation of 5-(Butylsulfinyl)-5,6-dihydro-2-[3-(trifluoromethyl)phenyl]-N-[4-(trifluoromethyl)phenyl]-4H-1,3,4-oxadiazine-4-carboxamide (Compound No. 15)

To an ice-cooled mixture of 1.1 g 5-(butylthio)-5,6-dihydro-2-[3-(trifluoromethyl)phenyl]-N-[4-(trifluoromethyl)phenyl]-4H-1,3,4-oxadiazine-4-carboxamide (Compound 14) and 1.8 g sodium bicarbonate in 15 ml dichloromethane was added dropwise a solution of 0.52 g 3-chloroperbenzoic acid in 4 ml dichloromethane. After stirring for 30 min the mixture was filtered. The filtrate was washed with 2M sodium sulfite and saturated sodium bicarbonate and then dried over sodium sulfate and concentrated under vacuum to produce 5-(butylsulfinyl)-5,6-dihydro-2-[3-(trifluoromethyl)phenyl]-N-[4-(trifluoromethyl)phenyl]-4H-1,3,4-oxadiazine-4-carboxamide. The structure was confirmed by 1H NMR.

### Example 15

### Preparation of 5-(butylsulfonyl)-5,6-dihydro-2-[3-(trifluoromethyl)phenyl]-N-[4-(trifluoromethyl)phenyl]-4H-1,3,4-oxadiazine-4-carboxamide (Compound No. 16)

To an ice-cool mixture of 1.1 g 5-(butylthio)-5,6-dihydro-2- [3- (trifluoromethyl)phenyl] -N- [4-(trifluoromethyl)phenyl]-4H-1,3,4-oxadiazine-4-carboxamide (Compound No. 14) and 1.8 g sodium bicarbonate in 15 ml dichloromethane was added 2.2 g 3-chloroperbenzoic acid. After stirring for 60 min the mixture was filtered. The filtrate was washed with 2M sodium sulfite and saturated sodium bicarbonate and then dried over sodium sulfate and concentrated under vacuum to produce 5-(butylsulfonyl)-5, 6-dihydro-2-[3-(trifluoromethyl)phenyl]-N-[4-(trifluoro-methyl)phenyl]-4H-1,3,4-oxadiazine-4-carboxamide. The structure was confirmed by 1H NMR.

Compounds 17 - 36 were prepared in a similar manner as described in Examples 13 - 15.

**TABLE 1**

| | | | | | | | |
|---|---|---|---|---|---|---|---|
| 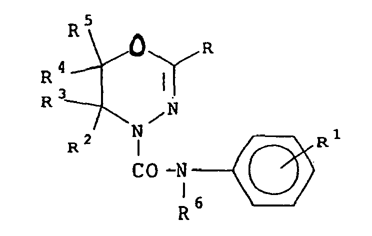 | | | | | | | |

| Cmpd No. | R | R¹ | R² | R³ | R⁴ | R⁵ | R⁶ |
|---|---|---|---|---|---|---|---|
| 1 | 3-CF₃C₆H₄ | 4-CF₃O | H | H | H | C₂H₅O | H |
| 2 | 4 -BrC₆H₄ | 4-CF₃O | H | H | H | C₂H₅O | H |
| 3 | 5-Br-2-C₄H₂S | 4-CF₃O | H | H | H | C₂H₅O | H |
| 4 | 5-Br-2-C₄H₂S | 4-CF₃O | H | H | CH₃ | CH₃O | H |
| 5 | 3-CF₃C₆H₄ | 4-CF₃O | CH₃ | H | H | C₂H₅O | H |
| 6¹ | 4-BrC₆H₄ | 4-CF₃O | H | CH₂ | H | CH₂O | H |
| 7² | 4-BrC₆H₄ | 4-CF₃O | H | CH₂CH₂ | H | CH₂O | H |
| 8 | 3-CF₃C₆H₄ | 4-CF₃O | H | H | H | CH₃O | H |
| 9 | 3-CF₃C₆H₄ | 4-CF₃ | H | H | H | C₂H₅O | H |
| 10 | 4-CF₃O- C₆H₄ | 4-CF₃ | H | H | H | CH₃ | H |
| 11 | 4-CF₃O- C₆H₄ | 4-CF₃ | H | H | H | 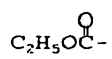 | H |
| 12 | 3-CF₃C₆H₄ | 4-CF₃O | H | H | H | C₂H₅O | CH₃ |
| 13 | 3-CF₃C₆H₄ | 4-CF₃O | H | H | H | C₂H₅O | 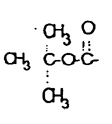 |

| | | | | | | | |
|---|---|---|---|---|---|---|---|
| ¹ R³ and R⁵ together form a 5-membered ring (furan) ² R³ and R⁵ together form a 6-membered ring (pyran) | | | | | | | |

**Table 1A**

| | | | | |
|---|---|---|---|---|
| 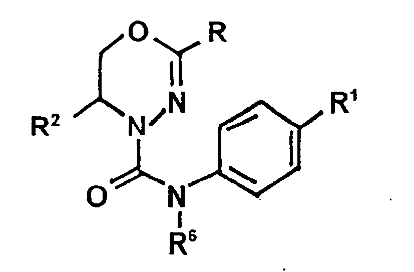 | | | | |

| Cmpd. No. | R | R¹ | R² | R⁶ |
|---|---|---|---|---|
| 14 | 3-CF₃C₆H₄ | 4-CF₃ | C₄H₉S | H |
| 15 | 3-CF₃C₆H₄ | 4-CF₃ | C₄H₉S=O | H |
| 16 | 3-CF₃C₆H₄ | 4-CF₃ | C₄H₉SO2 | H |
| 17 | 3-CF₃C₆H₄ | 4-CF₃ | C₆H₅S | H |
| 18 | 3-CF₃C₆H₄ | 4-CF₃ | C₆H₅S=O | H |
| 19 | 3-CF₃C₆H₄ | 4-CF₃ | C₆H₅SO2 | H |
| 20 | 3-CF₃C₆H₄ | 4-CF₃ | 3,5-di-CH₃-4- pyrazolyl | H |
| 21 | 3-CF₃C₆H₄ | 4-CF₃ | 2-thienyl | H |
| 22 | 3-CF₃C₆H₄ | 4-CF₃ | (CH₃0) ₂P=O | H |
| 23 | 3-CF₃C₆H₄ | 4-CF₃ | NC | H |
| 24 | 3-CF₃C₆H₄ | 4-CF₃ | 4-CH₃OC₆H₄ | H |
| 25 | 3-CF₃C₆H₄ | 4-CF₃ | HO | H |
| 26 | 3-CF₃C₆H₄ | 4-CF₃ | CF₃CH₂O | H |
| 27 | 3-CF₃C₆H₄ | 4-CF₃ | 2-oxo-cyclohexyl | H |
| 28 | 3-CF₃C₆H₄ | 4-CF₃ | CH₂=CHCH₂ | H |
| 29 | 3-CF₃C₆H₄ | 4-CF₃ | NO₂CH₂CH₂O | H |
| 30 | 3-CF₃C₆H₄ | 4-CF₃ | C₆H₅CH₂O | H |
| 31 | 3-CF₃C₆H₄ | 4-CF₃ | CH₃CO₂ | H |
| 32 | 3-CF₃C₆H₄ | 4-CF₃ | C₆H₅CO₂ | H |
| 33 | 3-CF₃C₆H₄ | 4-CF₃ | CH₃O | H |
| 34 | 3-CF₃C₆H₄ | 4-CF₃ | 5-methyl-2-furanyl | H |
| 35 | 3-CF₃C₆H₄ | 4-CF₃ | 2-furanyl | H |
| 36 | 3-CF₃C₆H₄ | 4-OCF₃ | 2-furanyl | H |

**TABLE 2**

| Compound No. | NMR Data (ppm) in DMSO |
|---|---|
| 1 | t(3) 1.2; m(4) 3.5-4.1; t (1) 5.7; m(8) 7.0-7.9; s(1) 9.4 |
| 2 | t (3) 1.2; m(4) 3.5-4.1; t (1) 5.4; m(8) 7.0-8.5; s (1) 8.4 |
| 3 | t (3) 1.2; m(4) 3.5-4.1; t (1) 5.4; m(8) 7.0-7.8; s (1) 8.3 |
| 4 | s(3) 1.5; d(1) 3.2; s(3) 3.4; q(1) 4.3; m(6) 6.9-7.8; s(1) 8.3 |
| 5 | t(3) 1.1; t(3) 1.3; q(2) 3.8; m(1) 4.4-4.8; d(1) 5.6; m(8) 7.1-8.2; s(1) 9.4 |
| 6 | m(2) 1.4-2.0; m(2) 3.5-3.9; m(1) 4.4-4.7; d(1) 5.7; m(8) 7.1-8.2; s(1) 9.4 |
| 7 | m(4) 1.4-2.2; m(2) 3.9-4.4; m(1) 4.8-5.2; d(1) 5.7; m(8) 7.1-8.2; s(1) 9.4 |
| 8 | s(3) 3.6; m(2) 3.3-4.3; t(1) 5.7; m(8) 7.1-8.6; s(1) 9.4 |
| 9 | t(3) 1.2; m(4) 3.5-4.3; t(1) 5.9; m(8) 7.5-8.6; s(1) 9.6 |
| 10 | d(3) 1.5; m(1) 3.3-3.6; m(2) 4.3-4.8; m(8) 7.5-8.3; s(1) 9.5 |
| 11 | t (3) 1.2; m(4) 3.9-4.6; t (1) 5.7; m(8) 7.4-8.3; s(1) 9.5 |
| 12 | t (3) 1.2; s (3) 3.3; m(4) 3.9-4.6; t (1) 5.7; m(8) 7.4-8.3 |
| 14 | s(1) 9.7; m(2) 8.5; m(6) 8.0-7.6; s(1) 6.1; s(2) 4.7; t(2) 2.8; m(4) 1.7-1.2; m(3) 0.9 |
| 15 | s(1) 9.8; m(2) 8.4; m(6) 8.0-7.6; s(1) 5.9; m(2) 5.1-4.6; m(2) 3.0; m(4) 1.8-1.5; m(3) 0.9 |
| 16 | s(1) 9.8; m(2) 8.4; m(6) 8.0-7.6; m(1) 6.2; d(1) 5.2; m(1) 4.6; m(2) 3.3; m(4) 1.8-1.3; m(3) 0.9 |
| 17 | s(1) 9.8; m(2) 8.5; m(11) 8.0-7.3; s(1) 6.3; s(2) 4.8 |
| 18 | s(1) 9.8; m(13) 8.5-7.5; d(1) 5.9; m(2) 5.3-4.6 |
| 19 | s(1) 9.6; m(2) 8.3; m(11) 7.9-7.5; m(1) 6.2; m(2) 5.3-4.6 |
| 20 | br s(1) 12.2; s(1) 9.6; m(2) 8.4; m(6) 8.0-7.6; s(1) 5.7; s(2) 4.6; s(6) 2.1 |
| 21 | s(1) 9.4; m(2) 8.3; m(9) 7.9-7.0; m(1) 6.1; m(2) 4.8 |
| 22 | s(1) 9.8; m(2) 8.4; m(6) 8.0-7.6; m(1) 5.3; m(2) 4.8; m(6) 3.7 |
| 23 | s(1) 9.8; m(2) 8.4; m(6) 7.9-7.6; s(1) 5.9; m(2) 5.2-4.5 |
| 24 | s(1) 9.7; m(2) 8.4; m(6) 8.0-7.6; d(2) 7.2; d(2) 7.8; s(1) 5.8; m(2) 4.7; s(3) 3.7 |
| 25 | s(1) 9.6; m(2) 8.4; m(6) 8.1-7.6; d(1) 6.7; d(1) 6.1; m(2) 4.5 |
| 26 | s(1) 9.7; m(2) 8.4; m(6) 8.0-7.6; s(1) 6.2; m(4) 4.9-4.1 |
| 27 | s(1) 9.6; m(2) 8.4; m(6) 8.2-7.7; m(1) 5.3; m(2) 4.7; m(1) 3.1; m(2) 2.6; m(6) 2.2-1.6 |
| 28 | s(1) 9.6; m(2) 8.4; m(6) 8.0-7.5; m(1) 5.8; m(5) 5.2-4.5; m(2) 2.4 |
| 29 | s(1) 9.6; m(2) 8.4; m(6) 8.1-7.6; d(1) 6.7; d(1) 6.1; m(2) 4.5 |
| 30 | s(1) 9.7; m(2) 8.4; m(6) 8.0-7.6; s(5) 7.3; s(1) 6.2; m(3) 4.8-4.3 |
| 31 | s(1) 9.8; m(2) 8.5; m(6) 8.0-7.6; s(1) 7.1; s(2) 4.7; s(3) 2.1 |
| 32 | s(1) 9.8; m(2) 8.5; m(12) 8.0-7.4; s(2) 4.8 |
| 33 | s(1) 9.7; m(2) 8.4; m(6) 8.0-7.6; s(1) 5.9; q(2) 4.5; s(3) 3.4 |
| 34* | s(1) 8.6; m(2) 8.1; m(6) 7.6; m(1) 6.2; m(2) 5.9; m(2) 5.0-4.3; s(3) 2.2 |
| 35* | s(1) 8.6; m(2) 8.2; m(7) 7.7-7.4; d(2) 6.3; m(1) 6.0; m(2) 4.7 |
| 36 | s(1) 9.3; m(2) 8.3; m(7) 7.7-7.1; d(2) 6.3; m(1) 6.0; m(2) 4.7 |

| | |
|---|---|
| * Spectrum taken in CDCl₃ | |

### Example A

### Stock Solution Preparation

The remaining examples relate to the insecticidal use of the compounds of this invention. In all these examples, a stock solution for the compounds was prepared at 1000 ppm by dissolving 0.13 gram of each compound to be tested in 13 ml of acetone and adding 117 ml of distilled water plus 5 drops of ethoxylated sorbitan monolaurate, a wetting agent. This stock solution was used in the remaining examples demonstrating the insecticidal use of representative compounds of this invention. For each example that follows, this stock solution was used and the specificized dilutions made. All the tests discussed below, which involved treatment with compounds of this invention were always repeated with controls, in which the active compound was not provided, to permit a comparison upon which the percent control was calculated.

### Example B

### Southern Corn Rootworm Test

The stock solution of 1000 ppm prepared in Example A above, was diluted to 100 ppm (test solution). For each compound, 2.5 ml of the test solution was pipetted onto a filter paper (Whatman #3) at the bottom of a 100 mm petri dish. Two corn seedlings were soaked in the 100 ppm solution for 1 hour and transferred to the petri dish containing the same test solution. After 24 hours, each dish was loaded with 5 second instar larvae of Southern Corn Rootworm (Diabrotica undecimpunctata). After five days, the number of live larvae was noted and the percent control, corrected by Abbott's formula [see J. Economic Entomology 18: 265-267 (1925)] was calculated.

The results or the testing of Southern Corn Rootworm (CR) are presented in Table 3 below.

### Example C

### Rice Planthopper Foliar Test

The stock solution of 1000 ppm prepared in Example A above, was used undiluted. One pot containing approximately 20 Mars variety rice seedlings was treated with each formulation by spraying with a spray atomizer. One day after treatment plants were covered with a tubular cage and twenty adult rice delphacids, Sogatodes orizicola, were transferred into each cage. Five days after transferring, counts were made of the surviving planthoppers in each pot and percent control was estimated.

Results of the testing of rice planthoppers (RPH) are presented in Table 3 below.

### Example D

### Tobacco Budworm Test

For each compound, 0.2 ml of the stock solution prepared in Example A above, was pipetted onto the surface of each of 5 diet cells, allowed to spread over the surfaces and air dried for two hours. Then a second instar Helicoverpa virescens larva was introduced into each cell. After 14 days, the number of living larvae was determined for each treatment and percent control, corrected by Abbott's formula, was calculated.

The results of the testing of tobacco budworms (TB) are presented in Table 3 below.

**TABLE 3**

| PERCENT CONTROL OF SOUTHERN CORN ROOTWORM, RICE PLANTHOPPER AND TOBACCO BUDWORM | | | |
|---|---|---|---|
| Compound No. | Percent Control | | |
| | CR | RPH | TB |
| 1 | 100 | 0 | 100 |
| 2 | 0 | 0 | 100 |
| 3 | 80 | 0 | 100 |
| 4 | 80 | 0 | 100 |
| 5 | 100 | 0 | 100 |
| 6 | 0 | 0 | 100 |
| 7 | 100 | 100 | 52 |
| 8 | 100 | 0 | 100 |
| 9 | 100 | 0 | 100 |
| 10 | 0 | 0 | 100 |
| 11 | 0 | 0 | 37 |
| 12 | 40 | 100 | 100 |
| 14 | 100 | -- | 25 |
| 15 | 100 | -- | 100 |
| 16 | 50 | -- | 100 |
| 17 | 47 | -- | 100 |
| 18 | 100 | -- | 100 |
| 19 | 100 | -- | 0 |
| 20 | 50 | -- | 100 |
| 21 | 100 | -- | 100 |
| 22 | 25 | -- | 100 |
| 23 | 100 | -- | 100 |
| 24 | 50 | -- | 100 |
| 25 | 60 | -- | 100 |
| 26 | 33 | -- | 100 |
| 27 | 100 | -- | 100 |
| 28 | 25 | -- | 100 |
| 29 | 100 | -- | 100 |
| 30 | 100 | -- | 100 |
| 31 | 100 | -- | 100 |
| 32 | 100 | -- | 100 |
| 33 | 100 | -- | 100 |
| 34 | 100 | -- | 100 |
| 35 | 80 | -- | 100 |
| 36 | 60 | -- | 100 |

## Claims

1. A compound having the formula: 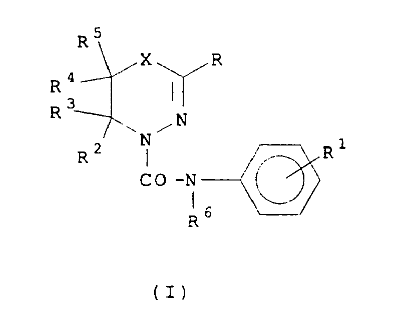 wherein
X is O, N or S;
R is phenyl, unsubstituted or mono-, di-, or tri-substituted with halogen, C₁-C₄ haloalkyl, C₁-C₄ haloalkoxy or C₁-C₄ haloalkylthio; or R is a C₄-C₅ heterocyclic group containing one nitrogen, sulfur, or oxygen atom, unsubstituted or mono-, di-, or tri-substituted with halogen, C₁-C₄ haloalkyl, or C₁-C₄ haloalkoxy;
R¹ is halogen, C₁-C₄ haloalkyl, C₁-C₄ haloalkoxy, C₁-C₄ haloalkyl sulfonyl, or C₁-C₄ haloalkyl sulfinyl;
R², R³, R⁴, and R⁵ are one of the following:
a) R², R³, and R⁴ are, independently, hydrogen, C₁-C₆ alkyl, C₁-C₆ alkoxy, or furanyl; and R⁵ is nitro, cyano, C₁-C₆ alkyl, di(C₁-C₆)alkylamino, C₁-C₆ alkylthio, C₁-C₆ alkoxy, C₁-C₆ haloalkoxy, phenoxy, phenylthio, or (C₁-C₆ alkoxy)carbonyl, wherein R³ and R⁵ together can form a ring; or
b) R³, R⁴, and R⁵ are hydrogen; and R² is C₁- C₆ alkylthio, C₁-C₆, alkylsulfinyl, C₁-C₆ alkylsulfonyl, nitro (C₁-C₆ alkoxy), hydroxy, di-(C₁-C₄ alkoxy)-phosphinyl, cyano, C₂-C₆ acyloxy, C₁-C₆ alkoxy, C₁-C₆ haloalkoxy, C₄-C₆ cycloalkyl, or C₃-C₆ alkenyl; or R is phenyl, phenylthio, phenylsulfinyl, phenylsulfonyl, pyrazolyl, furanyl, thienyl, phenyl (C₁-C₄ alkoxy), or benzoyloxy, unsubstituted or mono-, di-, or tri-substituted with halogen, C₁-C₄ alkyl, or C₁-C₄ alkoxy; and
R⁶ is hydrogen, C₁-C₆ alkyl, C₁-C₄ alkylthio, (C₁-C₄ alkoxy) C₁-C₄ alkyl, C₂-C₈ acyl, benzyl, or (C₁-C₆ alkoxy)-carbonyl.

2. A compound as recited in claim 1 having the formula:

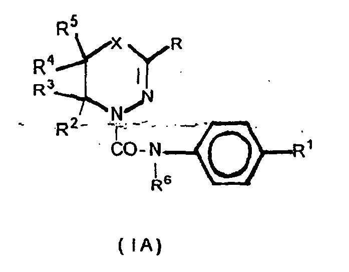

3. A compound as recited in claim 2 wherein R is phenyl, thienyl, furanyl, or pyridinyl, optionally mono-, di- or tri-substitutsd by bromo, chloro, C₁-C₄ alkyl, C₁-C₄ trihaloalkyl, or C₁-C₄ trihaloalkoxy.

4. A compound as recited in claim 3 wherein X is 0; R¹ is C₁-C₄ trihaloalkyl or C₁-C₄ trihaloalkoxy; R², R³, and R⁴ are, independently, C₁-C₄ alkyl or C₁-C₄ alkoxy; R⁵ is C₁-C₄ alkoxy, C₁-C₄ alkyl, C₁-C₄ alkylthio, or di (C₁-C₄)alkylamino; and R⁶ is hydrogen, C₁-C₄ alkyl, C₁-C₄ alkylthio, (C₁-C₄ alkoxy) methyl, or C₂-C₈ acyl.

5. A compound as recited in claim 4 wherein R is phenyl, thienyl, furanyl, or pyridinyl, optionally mono-substituted by one bromo, one chloro, one methyl, one t-butyl, one trihalomethyl, one trihaloethyl, one trihalomethoxy, or one trihaloethoxy.

6. A compound as recited in claim 5 wherein R¹ is trihalomethyl or trihaloethyl; R², R³, and R⁴ are, independently, methyl, ethyl, methoxy, or ethoxy; R⁵ is methoxy or ethoxy; and R⁶ is methyl, methylthio, methoxymethyl or acetyl.

7. A compound as recited in claim 6 wherein R is phenyl, thienyl, pyridinyl, or benzothienyl, substituted by bromo, chloro, methyl, t-butyl, trifluoromethyl, or trifluoromethoxy.

8. A compound as recited in claim 1 having the formula: 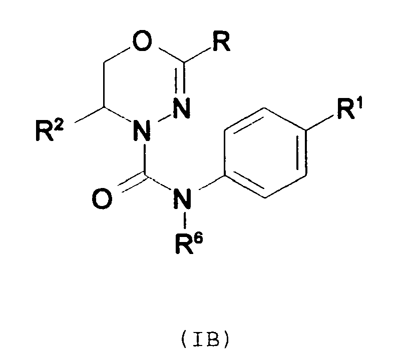 wherein
R is phenyl, unsubstituted or mono-, di-, or tri-substituted with halogen, C₁-C₄ haloalkyl, C₁-C₄ haloalkoxy, C₁-C₄ haloalkyl thio, C₁-C₄ haloalkyl sulfinyl, or C₁-C₄ haloalkyl sulfonyl; or R is a C₄-C₅ heterocyclic group containing one nitrogen, sulfur, or oxygen atom, unsubstituted or mono-, di-, or tri-substituted with halogen, C₁-C₄ haloalkyl, or C₁-C₄ haloalkoxy;
R¹ is halogen, C₁-C₄ haloalkyl, C₁-C₄ haloalkoxy, C₁-C₄ haloalkyl thio, C₁-C₆ haloalkyl sulfinyl, C₁-C₆ haloalkyl sulfonyl;
R² is C₁-C₆ alkylthio, C₁-C₆ alkylsulfinyl, C₁-C₆ alkylsulfonyl, nitro(C₁-C₆ alkoxy), hydroxy, di-(C₁-C₄ alkoxy)-phosphinyl, cyano, C₂-C₆ acyloxy, C₁-C₆ alkoxy, C₁-C₆ haloalkoxy, C₄-C₆ cycloalkyl, or C₃-C₆ alkenyl; or R² is phenyl, phenylthio, phenylsulfinyl, phenylsulfonyl, pyrazolyl, furanyl, thienyl, phenyl(C₁-C₄ alkoxy), or benzoyloxy, unsubstituted or mono-, di-, or tri-substituted with halogen, C₁-C₄ alkyl, or C₁-C₄ alkoxy; and
R⁶ is hydrogen, C₁-C₆ alkyl, (C₁-C₄ alkoxy) C₁-C₄ alkyl, C₂-C₆ haloacyl, C₂-C₈ acyl, or (C₁-C₆ alkoxy)carbonyl.

9. A compound as recited in claim 8 wherein R is phenyl, optionally mono-, di- or tri-substituted by bromo, chloro, C₁-C₄ alkyl, C₁-C₄ trihaloalkyl, or C₁-C₄ trihaloalkoxy.

10. A compound as recited in claim 9 wherein R¹ is C₁-C₄ trihaloalkyl or C₁-C₄ trihaloalkoxy; R² is C₁-C₆ alkylthio, C₁-C₆ alkylsulfinyl, C₁-C₆ alkylsulfonyl, nitro(C₁-C₄ alkoxy), hydroxy, di-(C₁-C₄ alkoxy)-phosphinyl, cyano, C₂-C₆ acyloxy, C₁-C₆ alkoxy, C₁-C₆ haloalkoxy, C₄-C₆ cycloalkyl, or C₃-C₆ alkenyl, phenyl, phenylthio, phenylsulfinyl, phenylsulfonyl, pyrazolyl, mono- or di-(C₁-C₄ alkyl)-pyrazolyl, furanyl, (C₁-C₄ alkyl)furanyl, thienyl, phenyl (C₁-C₄ alkoxy), or benzoyloxy; and R⁶ is hydrogen, C₁-C₄ alkyl, C₁-C₄ alkylthio, (C₁-C₄ alkoxy)-methyl, or C₂-C₈ acyl.

11. A compound as recited in claim 10 wherein R is phenyl, optionally mono-substituted by one bromo, one chloro, one methyl, one t-butyl, one trihalomethyl, one trihaloethyl, one trihalomethoxy, or one trihaloethoxy.

12. A compound as recited in claim 11 wherein R¹ is trihalomethyl or trihaloethyl; and R⁶ is hydrogen or methyl.

13. A compound as recited in claim 12 wherein R is phenyl substituted by bromo, chloro, methyl, t-butyl, trifluoromethyl, or trifluoromethoxy.

14. An insecticidal composition comprising: a) an effective amount of a compound as recited in claim 1; and b) a suitable carrier.

15. An insecticidal composition comprising: a) an effective amount of a compound as recited in claim 2; and b) a suitable carrier.

16. An insecticidal composition comprising: a) an effective amount of a compound as recited in claim 8; and b) a suitable carrier.

17. A method for controlling insects which comprises applying an effective amount of a compound as recited in claim 1 to the locus to be protected.

18. A method for controlling insects which comprises applying an effective amount of a compound as recited in claim 2 to the locus to be protected.

19. A method for controlling insects which comprises applying an effective amount of a compound as recited in claim 8 to the locus to be protected.

## Patentansprüche

1. Verbindung, welche die Formel: 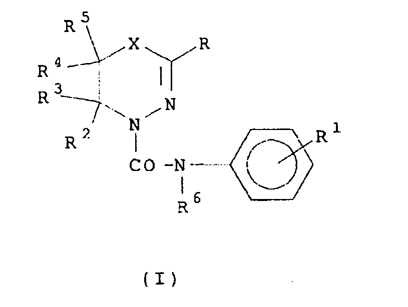 aufweist, wobei
X steht für O, N oder S;
R steht für Phenyl, das unsubstituiert oder mono-, di- oder trisubstituiert ist durch Halogen, C₁-C₄-Halogenalkyl, C₁-C₄-Halogenalkoxy oder C₁-C₄-Halogenalkylthio; oder R steht für eine heterocyclische C₄-C₅-Gruppe, die ein Stickstoff-, Schwefel- oder Sauerstoffatom enthält, die unsubstituiert oder mono-, di- oder trisubstituiert ist durch Halogen, C₁-C₄-Halogenalkyl oder C₁-C₄-Halogenalkoxy;
R¹ steht für Halogen, C₁-C₄-Halogenalkyl, C₁-C₄-Halogenalkoxy, C₁-C₄-Halogenalkylsulfonyl oder C₁-C₄-Halogenalkylsulfinyl;
R², R³, R⁴ und R⁵ für eine der folgenden Bedeutungen stehen:
a) R², R³ und R⁴ stehen unabhängig für Wasserstoff, C₁-C₆-Alkyl, C₁-C₆-Alkoxy oder Furanyl; und R⁵ steht für Nitro, Cyano, C₁-C₆-Alkyl, Di-(C₁-C₆)-alkylamino, C₁-C₆-Alkylthio, C₁-C₆-Alkoxy, C₁-C₆-Halogenalkoxy, Phenoxy, Phenylthio oder (C₁-C₆-Alkoxy)-carbonyl, wobei R³ und R⁵ zusammen einen Ring bilden können; oder
b) R³, R⁴ und R⁵ stehen für Wasserstoff; und R² steht für C₁-C₆-Alkylthio, C₁-C₆-Alkylsulfinyl, C₁-C₆-Alkylsulfonyl, Nitro-(C₁-C₆)-alkoxy, Hydroxy, Di-(C₁-C₄-alkoxy)-phosphinyl, Cyano, C₂-C₆-Acyloxy, C₁-C₆-Alkoxy, C₁-C₆-Halogenalkoxy, C₄-C₆-Cycloalkyl oder C₃-C₆-Alkenyl; oder R² steht für Phenyl, Phenylthio, Phenylsulfinyl, Phenylsulfonyl, Pyrazolyl, Furanyl, Thienyl, Phenyl-(C₁-C₄-alkoxy) oder Benzoyloxy, das unsubstituiert oder mono-, di- oder trisubstituiert ist durch Halogen, C₁-C₄-Alkyl oder C₁-C₄-Alkoxy; und
R⁶ steht für Wasserstoff, C₁-C₆-Alkyl, C₁-C₄-Alkylthio, (C₁-C₄-Alkoxy)-C₁-C₄-alkyl, C₂-C₈-Acyl, Benzyl oder (C₁-C₆-Alkoxy)-carbonyl.

2. Verbindung nach Anspruch 1, welche die Formel: 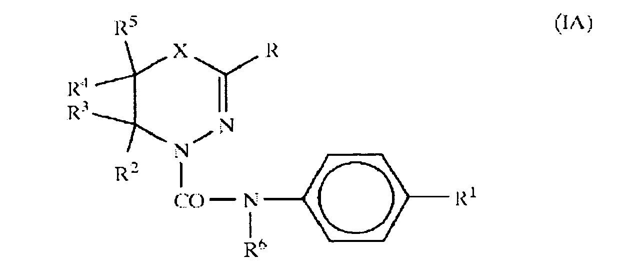 aufweist.

3. Verbindung nach Anspruch 2, wobei R für Phenyl, Thienyl, Furanyl oder Pyridinyl, gegebenenfalls mono-, di- oder trisubstituiert durch Brom, Chlor, C₁-C₄-Alkyl, C₁-C₄-Trihalogenalkyl oder C₁-C₄-Trihalogenalkoxy, steht.

4. Verbindung nach Anspruch 3, wobei X für O steht; R¹ für C₁-C₄-Trihalogenalkyl oder C₁-C₄-Trihalogenalkoxy steht; R², R³ und R⁴ unabhängig stehen für C₁-C₄-Alkyl oder C₁-C₄-Alkoxy: R⁵ für C₁-C₄-Alkoxy, C₁-C₄-Alkyl, C₁-C₄-Alkylthio oder Di-(C₁-C₄)-alkylamino steht; und R⁶ für Wasserstoff, C₁-C₄-Alkyl, C₁-C₄-Alkylthio, (C₁-C₄-Alkoxy)-methyl oder C₂-C₈-Acyl steht.

5. Verbindung nach Anspruch 4, wobei R steht für Phenyl, Thienyl, Furanyl oder Pyridinyl, gegebenenfalls monosubstituiert durch ein Brom, ein Chlor, ein Methyl, ein tert.-Butyl, ein Trihalogenmethyl, ein Trihalogenethyl, ein Trihalogenmethoxy oder ein Trihalogenethoxy.

6. Verbindung nach Anspruch 5, wobei R¹ steht für Trihalogenmethyl oder Trihalogenethyl; R², R³ und R⁴ unabhängig stehen für Methyl, Ethyl, Methoxy oder Ethoxy; R⁵ steht für Methoxy oder Ethoxy; und R⁶ steht für Methyl, Methylthio, Methoxymethyl oder Acetyl.

7. Verbindung nach Anspruch 6, wobei R steht für Phenyl, Thienyl, Pyridinyl oder Benzothienyl, substituiert durch Brom, Chlor, Methyl, tert.-Butyl, Trifluormethyl oder Trifluormethoxy.

8. Verbindung nach Anspruch 1, welche die Formel: 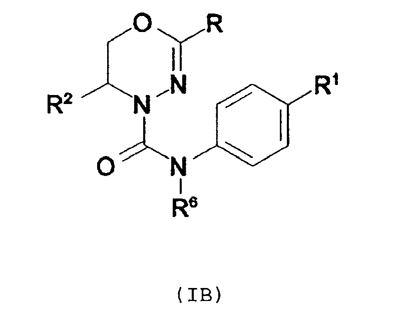 aufweist, wobei
R steht für Phenyl, das unsubstituiert oder mono-, di- oder trisubstituiert ist durch Halogen, C₁-C₄-Halogenalkyl, C₁-C₄-Halogenalkoxy, C₁-C₄-Halogenalkylthio, C₁-C₄-Halogenalkylsulfinyl oder C₁-C₄-Halogenalkylsulfonyl; oder R steht für eine heterocyclische C₄-C₅-Gruppe, die ein Stickstoff-, Schwefel- oder Sauerstoffatom enthält, die unsubstituiert oder mono-, di- oder trisubstituiert ist durch Halogen, C₁-C₄-Halogenalkyl oder C₁-C₄-Halogenalkoxy;
R¹ steht für Halogen, C₁-C₄-Halogenalkyl, C₁-C₄-Halogenalkoxy, C₁-C₄-Halogenalkylthio, C₁-C₆-Halogenalkylsulfinyl, C₁-C₆-Halogenalkylsulfonyl;
R² steht für C₁-C₆-Alkylthio, C₁-C₆-Alkylsulfinyl, C₁-C₆-Alkylsulfonyl, Nitro-(C₁-C₆-alkoxy), Hydroxy, Di-(C₁-C₄-alkoxy)-phosphinyl, Cyano, C₂-C₆-Acyloxy, C₁-C₆-Alkoxy, C₁-C₆-Halogenalkoxy, C₄-C₆-Cycloalkyl oder C₃-C₆-Alkenyl; oder R² steht für Phenyl, Phenylthio, Phenylsulfinyl, Phenylsulfonyl, Pyrazolyl, Furanyl, Thienyl, Phenyl-(C₁-C₄-alkoxy) oder Benzoyloxy, unsubstituiert oder mono-, di- oder trisubstituiert durch Halogen, C₁-C₄-Alkyl oder C₁-C₄-Alkoxy; und
R⁶ steht für Wasserstoff, C₁-C₆-Alkyl, (C₁-C₄-Alkoxy)-C₁-C₄-alkyl, C₂-C₆-Halogenacyl, C₂-C₈-Acyl oder (C₁-C₆-Alkoxy)-carbonyl.

9. Verbindung nach Anspruch 8, wobei R für Phenyl steht, das gegebenenfalls mono-, di- oder trisubstituiert ist durch Brom, Chlor, C₁-C₄-Alkyl, C₁-C₄-Trihalogenalkyl oder C₁-C₄-Trihalogenalkoxy.

10. Verbindung nach Anspruch 9, wobei R¹ steht für C₁-C₄-Trihalogenalkyl oder C₁-C₄-Trihalogenalkoxy; R² steht für C₁-C₆-Alkylthio, C₁-C₆-Alkylsulfinyl, C₁-C₆-Alkylsulfonyl, Nitro-(C₁-C₄-alkoxy), Hydroxy, Di-(C₁-C₄-alkoxy)-phosphinyl, Cyano, C₂-C₆-Acyloxy, C₁-C₆-Alkoxy, C₁-C₆-Halogenalkoxy, C₄-C₆-Cycloalkyl oder C₃-C₆-Alkenyl, Phenyl, Phenylthio, Phenylsulfinyl, Phenylsulfonyl, Pyrazolyl, Mono- oder Di-(C₁-C₄-alkyl)-pyrazolyl, Furanyl, (C₁-C₄-Alkyl)-furanyl, Thienyl, Phenyl-(C₁-C₄-alkoxy) oder Benzoyloxy; und R⁶ steht für Wasserstoff, C₁-C₄-Alkyl, C₁-C₄-Alkylthio, (C₁-C₄-Alkoxy)-methyl oder C₂-C₈-Acyl.

11. Verbindung nach Anspruch 10, wobei R für Phenyl steht, das gegebenenfalls monosubstituiert ist durch ein Brom, ein Chlor, ein Methyl, ein tert.-Butyl, ein Trihalogenmethyl, ein Trihalogenethyl, ein Trihalogenmethoxy oder ein Trihalogenethoxy.

12. Verbindung nach Anspruch 11, wobei R¹ für Trihalogenmethyl oder Trihalogenethyl steht; und R⁶ für Wasserstoff oder Methyl steht.

13. Verbindung nach Anspruch 12, wobei R für Phenyl steht, das substituiert ist durch Brom, Chlor, Methyl, tert.-Butyl, Trifluormethyl oder Trifluormethoxy.

14. Insektizidzusammensetzung, umfassend: a) eine wirksame Menge einer Verbindung gemäß Anspruch 1 und b) einen geeigneten Träger.

15. Insektizidzusammensetzung, umfassend: a) eine wirksame Menge einer Verbindung gemäß Anspruch 2 und b) einen geeigneten Träger.

16. Insektizidzusammensetzung, umfassend: a) eine wirksame Menge einer Verbindung gemäß Anspruch 8 und b) einen geeigneten Träger.

17. Verfahren zur Bekämpfung von Insekten, das umfasst ein Aufbringen einer wirksamen Menge einer Verbindung gemäß Anspruch 1 auf den Ort, der geschützt wird.

18. Verfahren zur Bekämpfung von Insekten, das umfasst ein Aufbringen einer wirksamen Menge einer Verbindung gemäß Anspruch 2 auf den Ort, der geschützt wird.

19. Verfahren zur Bekämpfung von Insekten, das umfasst ein Aufbringen einer wirksamen Menge einer Verbindung gemäß Anspruch 8 auf den Ort, der geschützt wird.

## Revendications

1. Composé présentant la formule : 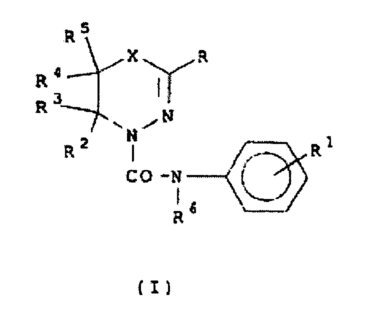 dans laquelle
X représente O, N ou S,
R représente un groupe phényle non substitué ou mono-, di- ou trisubstitué avec un atome d'halogène, un groupe halogénoalkyle en C₁ à C₄, un groupe halogénoalcoxy en C₁ à C₄ ou un groupe halogénoalkylthio en C₁ à C₄, ou R représente un groupe hétérocyclique en C₄ à C₅ qui contient un atome d'azote, un atome de soufre ou un atome d'oxygène, non substitué ou mono-, di- ou trisubstitué avec un atome d'halogène, un groupe halogénoalkyle en C₁ à C₄ ou un groupe halogénoalcoxy en C₁ à C₄,
R¹ représente un atome d'halogène, un groupe halogénoalkyle en C₁ à C₄, un groupe halogénoalcoxy en C₁ à C₄, un groupe halogéno(alkyle en C₁ à C₄) sulfonyle ou un groupe halogéno(alkyle en C₁ à C₄)sulfinyle,
R², R³ R⁴ et R⁵ représentent l'un des groupes suivants :
a) R², R³ et R⁴ représentent indépendamment un hydrogène, un groupe alkyle en C₁ à C₆, un groupe alcoxy en C₁ à C₆ ou un groupe furanyle et R⁵ représente un groupe nitro, un groupe cyano, un groupe alkyle en C₁ à C₆, un groupe di(alkyle en C₁ à C₆)amino, un groupe (alkyle en C₁ à C₄)thio, un groupe alcoxy en C₁ à C₆, un groupe halogéno(alcoxy en C₁ à C₆), un groupe phénoxy, un groupe phénylthio ou un groupe (alcoxy en C₁ à C₆)carbonyle, R³ et R⁵ pouvant former ensemble un groupe cycle ou
b) R³, R⁴ et R⁵ représentent l'hydrogène et R² représente un groupe (alkyle en C₁ à C₆)thio, un groupe (alkyle en C₁ à C₆) sulfinyle, un groupe (alkyle en C₁ à C₄) sulfonyle, un groupe nitro (alcoxy en C₁ à C₆), un groupe hydroxyle, un groupe di(alcoxy en C₁ à C₄) phosphinyle, un groupe cyano, un groupe acyloxy en C₁ à C₆, un groupe alcoxy en C₁ à C₆, un groupe halogénoalcoxy en C₁ à C₆, un groupe cyclo (alkyle en C₁ à C₆) ou un groupe alcényle en C₁ à C₆, ou R² représente un groupe phényle, un groupe phénylthio, un groupe phénylsulfinyle, un groupe phénylsulfonyle, un groupe pyrazolyle, un groupe furanyle, un groupe thiényle, un groupe phényl(alcoxy en C₁ à C₄) ou un groupe benzoyloxy, non substitué ou mono-, di- ou trisubstitués avec un atome d'halogène, un groupe alkyle en C₁ à C₄ ou un groupe alcoxy en C₁ à C₄ et
R⁶ représente l'hydrogène, un groupe alkyle en C₁ à C₆, un groupe (alkyle en C₁ à C₄)thio, un groupe (alcoxy en C₁ à C₄)alkyle en C₁ à C₄, un groupe acyle en C₂ à C₈, un groupe benzyle ou un groupe (alcoxy en C₁ à C₆)carbonyle.

2. Composé selon la revendication 1, de formule :

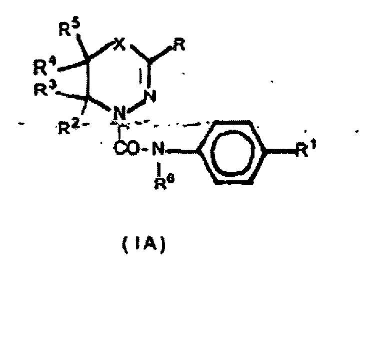

3. Composé selon la revendication 2, dans lequel R représente un groupe phényle, un groupe thiényle, un groupe furanyle, un groupe pyridinyle, facultativement mono-, di- ou trisubstitués avec un atome de brome, un atome de chlore, un groupe alkyle en C₁ à C₄, un groupe trihalogéno (alkyle en C₁ à C₄) ou un groupe trihalogéno(alcoxy en C₁ à C₄).

4. Composé selon la revendication 3, dans lequel X représente O, R¹ représente un groupe trihalogéno(alkyle en C₁ à C₄) ou un groupe trihalogéno(alcoxy en C₁ à C₄), R² R³ et R⁴ représentant indépendamment un groupe alkyle en C₁ à C₄ ou un groupe alcoxy en C₁ à C₄, R⁵ représente un groupe alcoxy en C₁ à C₄, un groupe alkyle en C₁ à C₄, un groupe (alkyle en C₁ à C₄) thio ou un groupe di(alkyle en C₁ à C₄)amino et R⁶ représente l'hydrogène, un groupe alkyle en C₁ à C₄, un groupe (alkyle en C₁ à C₄) thio, un groupe (alcoxy en C₁ à C₄)méthyle ou un groupe acyle en C₂ à C₈.

5. Composé selon la revendication 4, dans lequel R représente le phényle, un groupe thiényle, un groupe furanyle ou un groupe pyridinyle, facultativement monosubstitués avec un atome de brome, un atome de chlore, un groupe méthyle, un groupe t-butyle, un groupe trihalogénométhyle, un groupe trihalogénoéthyle, un groupe trihalogénométhoxy ou un groupe trihalogénoéthoxy.

6. Composé selon la revendication 4, dans lequel R¹ représente un groupe trihalogénométhyle ou un groupe trihalogénoéthyle, R³, R³ et R⁴ représentent indépendamment un groupe méthyle, un groupe éthyle, un groupe méthoxy ou un groupe éthoxy, R⁵ représente un groupe méthoxy ou un groupe éthoxy et R⁶ représente un groupe méthyle, un groupe méthylthio, un groupe méthoxyméthyle ou un groupe acétyle.

7. Composé selon la revendication 6, dans lequel R représente un groupe phényle, un groupe thiényle, un groupe pyridinyle ou un groupe benzothiényle, substitués avec un atome de brome, un atome de chlore, un groupe méthyle, un groupe t-butyle, un groupe trifluorométhyle ou un groupe trifluorométhoxy.

8. Composé selon la revendication 1, de formule : 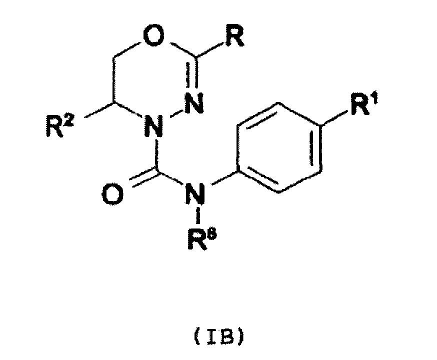 dans laquelle
R représente un groupe phényle non substitué ou mono-, di- ou trisubstitué avec un atome d'halogène, un groupe halogénoalkyle en C₁ à C₄, un groupe halogénoalcoxy en C₁ à C₄ ou un groupe halogénoalkylthio en C₁ à C₄, ou R représente un groupe hétérocyclique en C₄ à C₅ qui contient un atome d'azote, un atome de soufre ou un atome d'oxygène, non substitué ou mono-, di- ou trisubstitué avec un atome d'halogène, un groupe halogénoalkyle en C₁ à C₄ ou un groupe halogénoalcoxy en C₁ à C₄,
R¹ représente un atome d'halogène, un groupe halogénoalkyle en C₁ à C₄, un groupe halogénoalcoxy en C₁ à C₄, un groupe halogéno(alkyle en C₁ à C₄)thio, un groupe halogéno(alkyle en C₁ à C₄)sulfinyle ou un groupe halogéno(alkyle en C₁ à C₄)sulfonyle,
R² représente un groupe (alkyle en C₁ à C₆)thio, un groupe (alkyle en C₁ à C₆)sulfinyle, un groupe (alkyle en C₁ à C₄)sulfonyle, un groupe nitro(alcoxy en C₁ à C₆), un groupe hydroxyle, un groupe di(alcoxy en C₁ à C₄)phosphinyle, un groupe cyano, un groupe acyloxy en C₁ à C₆, un groupe alcoxy en C₁ à C₆, un groupe halogénoalcoxy en C₁ à C₆, un groupe cyclo(alkyle en C₁ à C₆) ou un groupe alcényle en C₁ à C₆, ou R² représente un groupe phényle, un groupe phénylthio, un groupe phénylsulfinyle, un groupe phénylsulfonyle, un groupe pyrazolyle, un groupe furanyle, un groupe thiényle, un groupe phényl(alcoxy en C₁ à C₄) ou un groupe benzoyloxy, non substitué ou mono-, di- ou trisubstitués avec un atome d'halogène, un groupe alkyle en C₁ à C₄ ou un groupe alcoxy en C₁ à C₄ et
R⁶ représente l'hydrogène, un groupe alkyle en C₁ à C₆, un groupe (alcoxy en C₁ à C₄)alkyle en C₁ à C₄, un groupe halogénoacyle en C₂ à C₈, un groupe halogénoacyle en C₂ à C₈, un groupe benzyle ou un groupe (alcoxy en C₁ à C₆)carbonyle.

9. Composé selon la revendication 8, dans lequel R représente un groupe phényle, facultativement mono-, di- ou trisubstitué avec un atome de brome, un atome de chlore, un groupe alkyle en C₁ à C₄), un groupe trihalogéno (alkyle en C₁ à C₄) ou un groupe trihalogéno(alcoxy en C₁ à C₄).

10. Composé selon la revendication 9, dans lequel R¹ représente un groupe trihalogéno(alkyle en C₁ à C₄) ou trihalogéno(alcoxy en C₁ à C₄), R² représente un groupe (alkyle en C₁ à C₆)thio, un groupe (alkyle en C₁ à C₆)sulfinyle, un groupe (alkyle en C₁ à C₆)sulfonyle, un groupe nitro(alcoxy en C₁ à C₄), un groupe hydroxy, un groupe di(alcoxy en C₁ à C₄)phosphinyle, un groupe cyano, acyloxy en C₂ à C₆), un groupe halogénoalcoxy en C₁ à C₆, cycloalkyle en C₁ à C₆ ou un groupe alcényle en C₃ à C₄, un groupe phényle, un groupe phénylthio, un groupe phénylsulfinyle, un groupe phénylsulfonyle, un groupe pyrazolyle, un groupe mono- ou di(alkyle en C₁ à C₄) pyrazolyle, un groupe furanyle, un groupe (alkyle en C₁ à C₄)furanyle, un groupe thiényle, un groupe phényl (alcoxy en C₁ à C₄) ou un groupe benzoyloxy et R⁶ représente l'hydrogène, un groupe alkyle en C₁ à C₄, un groupe alkylthio en C₁ à C₄, un groupe (alcoxy en C₁ à C₄)méthyle ou un groupe acyle en C₂ à C₈.

11. Composé selon la revendication 10, dans lequel R représente un groupe phényle, facultativement monosubstitué avec un atome de brome, un atome de chlore, un groupe méthyle, un groupe t-butyle, un groupe triahalogénométhyle, un groupe trihalogénoéthyle, un groupe trihalogénométhoxy ou un groupe trihalogénoéthoxy.

12. Composé selon la revendication 11, dans lequel R¹ représente un groupe trihalogénométhyle ou un groupe trihalogénoéthyle et R⁶ représente l'hydrogène ou un groupe méthyle.

13. Composé selon la revendication 12, dans lequel R représente un groupe phényle substitué avec un atome de brome, un atome de chlore, un groupe méthyle, un groupe t-butyle, un groupe trifluorométhyle ou un groupe trifluorométhoxy.

14. Composition insecticide qui comprend : a) une quantité efficace d'un groupe composé selon la revendication 1 et b) un agent porteur approprié.

15. Composition insecticide qui comprend : a) une quantité efficace d'un composé selon la revendication 2 et b) un agent porteur approprié.

16. Composition insecticide qui comprend : a) une quantité efficace d'un composé selon la revendication 8 et b) un agent porteur approprié.

17. Procédé de contrôle des insectes qui comprend l'application d'une quantité efficace d'un composé selon la revendication 1 sur le site à protéger.

18. Procédé de contrôle des insectes qui comprend l'application d'une quantité efficace d'un composé selon la revendication 2 sur le site à protéger.

19. Procédé de contrôle des insectes qui comprend l'application d'une quantité efficace d'un composé selon la revendication 8 sur le site à protéger.
